# EUROPEAN PATENT SPECIFICATION

(11) **EP 1 797 066 B1**
(45) Date of publication and mention of the grant of the patent: **12.11.2014**
(21) Application number: 05786862.2
(22) Date of filing: 06.09.2005
(51) Int. Cl.: C07D 341/00, A23L 1/226

(54) **DISUBSTITUTED TETRATHIANES AS FRAGRANCES OR FLAVOURINGS**
DISUBSTITUIERTE TETRATHIANE ALS DUFT- ODER AROMASTOFFE
TETRATHIANES DISUBSTITUEES UTILISEES COMME PARFUMS OU COMME AROMES

(30) Priority: 10.09.2004 US 609034 P
(43) Date of publication of application: 20.06.2007
(73) Proprietor: Symrise AG, 37603 Holzminden (DE)
(72) Inventor: OTT, Frank, 37603 Holzminden (DE); KINDEL, Günter, 37671 Höxter (DE); LEY, Jakob, 37603 Holzminden (DE); KRAMMER, Gerhard, 37603 Holzminden (DE); SCHMIDT, Claus-Oliver, 37603 Holzminden (DE); SCHIEBERLE, Peter, 85354 Freising (DE); GRANVOGL, Michael, 80997 München (DE)
(74) Representative: Fabry, Bernd
(86) International application number: PCT/EP2005/054380
(87) International publication number: WO 2006/027352

(56) References cited:
- US-A- 3 503 758
- US-A- 4 259 507
- US-A- 4 263 331
- US-A- 4 293 579
- C.-Y. TAI, C.-T. HO: "Influence of Cysteine Oxidation on Thermal Formation of Maillard Aromas" J. AGRIC. FOOD. CHEM., vol. 45, no. 9, 1997, pages 3586-3589, XP002354495
- L. N. NIXON ET AL.: "Nonacidic Constitutents of Volatiles from Cooked Mutton" J. AGRIC. FOOD CHEM., vol. 27, no. 2, 1979, pages 355-359, XP002354496
- C.-K. SHU ET AL.: "Volatile Components of the Thermal Degradation of Cystine in Water" J. AGRIC. FOOD CHEM., vol. 33, no. 3, 1985, pages 438-442, XP002354497
- C.-K. SHU ET AL.: "pH Effect on the Volatile Components in the Thermal Degradation of Cysteine" J. AGRIC. FOOD CHEM., vol. 33, no. 3, 1985, pages 442-446, XP002354498
- T.-H. YU ET AL.: "Volatile Compounds from Thermal Degradation of Alliin and Deoxyalliin in an Aqueous Solution" J. AGRIC. FOOD CHEM., vol. 42, no. 1, 1994, pages 146-153, XP002354499
- W. RÖDEL, H.-P. KRUSE: "Sensorische Eigenschaften der cis/trans-Isomeren von 3,5-Di-n-alkyl-1,2,4-trithiolanen" DIE NAHRUNG, vol. 26, no. 4, 1982, pages 377-383, XP009057213
- W. FRANEK: "1,2,4,5-Tetrathiane - II. Strukturelle und spektroskopische Eigenschaften, Aromaforschung, Anwendungen" SULFUR REPORTS, vol. 10, no. 3, 1991, pages 233-272, XP009057215
- PATENT ABSTRACTS OF JAPAN vol. 1996, no. 09, 30 September 1996 (1996-09-30) & JP 08 116914 A (T. HASEGAWA CO., LTD.), 14 May 1996 (1996-05-14)
- LEDL F: "Analyse eines synthetischen Zwiebelaromas", ZEITSCHRIFT FUER LEBENSMITTELUNTERSUCHUNG UND -FORSCHUNG, vol. 157, 1 January 1975 (1975-01-01), pages 28-33, XP009165747, ISSN: 0044-3026

## Description

### Field of invention

The invention relates
- to mixtures comprising tetrathianes as well as disubstituted trithiolanes and/or disubstituted pentathiepanes,
- to corresponding methods of producing, enhancing or modifying a taste and/or odour,
- to fragrance, flavouring or taste-imparting substance compositions comprising an above- mentioned mixture,
- to corresponding preparations, and
- to processes for the preparation of an above-mentioned mixture.

### State of the art

A large number of *Liliaceae* of the genus *Allium* are used for consumption. In chemical terms, these plants are distinguished by the fact that their characteristic taste and odour properties are for the most part caused by organic substances which contain one or more sulphur atoms.

A characteristic flavouring of raw, freshly cut Allium plants is allicin (allylthioallyl sulfinate). Allicin is an unstable compound which is converted after a short time into sulphur-containing, open-chained allyl derivatives (as in garlic) or 1-propenyl derivatives (as in onion).

When Allium plants are heated (boiling, roasting, frying, etc.), the enzymatic process of conversion of the allicin is greatly accelerated. In addition, sweet taste impressions occur to an increased extent as a result of the thermal degradation of saccharide. Maillard reactions and other chemical processes lead to the formation of flavourings which impart interesting notes to many dishes. In addition to the allyl and 1-propenyl derivatives mentioned above, cyclic sulphur-containing compounds inter alia are also formed **(**Studies in Natural Products Chemistry, 2000, Volume 23, 455-485**).**

In particular, the typical representatives of freshly cut onions, the 1-propenyl sulfide derivatives, are desirable flavourings, but they are obtainable chemically only with great difficulty. In addition, even when these compounds are success fully prepared and isolated, they exhibit only very low chemical stability.

For the mentioned reasons it is desirable to provide a substance or mixture of substances that has a taste and/or odour impression of Allium species or culinary preparations thereof and that is capable of imparting such a taste and/or odour impression to products, in particular to non-perishable foodstuffs or (semi-finished products suitable for consumption.

Hitherto it has not been possible to imitate the typical taste and/or odour impression of fresh, or freshly cut, or roasted or fried onions using the existing flavourings while retaining these sensory impressions over a prolonged period.

The object of the present invention was, therefore, to provide such a flavouring or mixture of flavourings. The flavouring or mixture of flavourings should be readily obtainable and, in particular, should exhibit high chemical stability or stability to storage.

### Description of the invention

A first object of the present invention refers to a mixture comprising a compound of formula **2** and a compound of formula **1** as well as, optionally, a compound of formula **3** 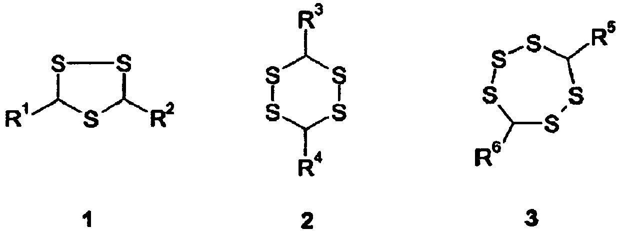 wherein R¹, R², R³, R⁴, R⁵ and R⁶ independently of one another represent a C₂-C₄-alkyl or - alkenyl radical and wherein the weight ratio compound of formula **2 :** compound of formula **1** is greater than or equal to 1: 5, preferably greater than or equal to 1: 4.

The invention relates also to a mixture comprising a compound of formula **2** and a compound of formula **3** as well as, optionally, a compound of formula **1** wherein R¹, R², R³, R⁴, R⁵ and R⁶ independently of one another represent a C₂-C₄-alkyl or -alkenyl radical.

### Tetrathianes, trithiolanes and pentathiepanes

For the sake of simplicity, the 3,6-disubstituted 1,2,4,5-tetrathianes of formula **2** to be used according to the invention are referred to as tetrathianes, the 3,5- disubstituted 1,2,4-trithiolanes of formula 1 are referred to as trithiolanes and the 4,7-disubstituted 1,2,3,5, 6-pentathiepanes of formula 3 are referred to as pentathiepanes.

The alkyl and alkenyl radicals within the scope of the invention may be straight- or branched-chained. The alkyl and/or alkenyl radicals on the ring in the com pounds of formulae **1** to **3** may have the cis or trans-configuration. Where applicable, the double bond in the alkenyl radical may have the E or Z configuration.

Suitable alkyl radicals are in particular: ethyl, 1 -propyl, 2-propyl, 1 -butyl, 2-butyl, 2-methyl-1-propyl and 2-methyl-2-propyl.

Suitable alkenyl radicals are in particular: 1-ethenyl, E/Z-1-propenyl, 2-propenyl, E/Z-1-butenyl, E/Z-2-butenyl, E/Z-2-methyl-1-propenyl and 2-methyl-2-propenyl. In formulae 1, 2 and 3, the radicals R¹, R² or R³, R⁴ or R⁵, R⁶ each independently of the others preferably represents a C₂-C₄-alkyl radical, particularly preferably the same C₂-C₄-alkyl radical. Very particularly preferably, R¹, R² or R³, R⁴ or R⁵, R⁶ each represents ethyl.

In Z. Lebensm. Unters.-Forsch. Vol. 157, 28-33 (1975**),** the reaction of propionaldehyde with ammonia and hydrogen sulfide is described. The synthetic onion flavour resulting therefrom, which comprised a plurality of compounds, has been investigated by means of gas chromatography and mass spectrometry. It contained inter alia 3,5 -diethyl-1,2,4-trithiolane (formula 1, R¹ and R² = ethyl) and 3,6- diethyl-1,2,4,5-tetrathiane (formula 2, R³ and R⁴ = ethyl). However, neither 3,5- diethyl-1,2,4-trithiolane nor 3,6-diethyl-1,2,4,5-tetrathiane were recognised as being sensorial effective components or components valuable in terms of odour and/or taste. The ratio of diethyltrithiolane to diethyltetrathiane, which can be estimated from the gas chromatogram of this mixture indicated in the mentioned document, is approximately 6 - 7: 1.

In Wein-Wiss., Vol. 48(3-6), 214-218 (1993**),** 3,6-dimethyl-1,2,4,5-tetrathiane (compound of formula **2** not to be used according to the invention wherein R³ and R⁴ = methyl) is described in sensory terms as rubbery, chemical and sulphurous, and 4,7-dimethyl-1,2,3,5,6-pentathiepane (compound of formula **3** not to be used according to the invention wherein R⁵ and R⁶ = methyl) is described as meaty. By combining these two compounds together with 3,5-dimethyl-1,2,4-trithiolane (compound of formula 1 not to be used according to the invention, R¹ and R² = methyl) it is possible according to the general statements made by the authors to achieve different impressions such as onion, rubber, garlic or cabbage, synergistic or antagonistic effects being said to occur depending on the concentration of the substance in question.

Many trithiolanes of formula 1 wherein R¹, R² = C₂-C₄-alkyl which are suitable for use in mixtures according to the invention have been identified predominantly in various meat flavours as well as in yeast extracts and in some cases have been described in sensory terms, and have been cited in the state of the art¹²³.
- Accordingly, 3,5-diethyl-1,2,4-trithiolane (formula 1, R³ and R⁴ = ethyl) is described in sensory terms as sulphurous, rubbery, tarragon- and fennel-like, 3-ethyl-5-n-propyl-1,2,4-trithiolane (formula 1, R³ = ethyl, R⁴ = n-propyl) is described as fatty, tarragon-and fennel- like, and 3,5-di-n-butyl-1,2,4-trithiolane (formula 1, R³ and R⁴ = n-butyl) is described as fatty, tallow- and crackling-like. Own test of the applicant on pure 3-ethyl-5- isopropyl-1,2,4-trithiolane (formula 1, R³ = ethyl, R⁴ = isopropyl) have shown that it has a markedly sulphurous, slightly burnt leek character.
- 3,5-Diisopropyl-1,2,4-trithiolane (formula 1, R¹ and R² = isopropyl) (purity greater than 95 percent) can be described as follows: garlic, fatty-oily, mustard, onion, sulphurous.
- 3,5-Diisobutyl-1,2,4-trithiolane (formula 1, R¹ and R² = isobutyl) (purity greater than 95 percent) can be described as follows: bacon, fatty-oily, egg, dripping with crackling, roasted, rubber.
111th International Congress of Essential Oils, Fragrances and Flavours, New Dehli, India, Proceedings, Vol. 4, 215-243 (1989 )
² Mikrobiol. Technol. Lebensm. Vol. 13, 30-57 (1991)
³ Proceedings on the 3rd International Haarmann and Reimer Symposium on Recent Developments in Flavour and Fragrance Chemistry, Kyoto, Japan, 183-213 (1992)

A synthesis route for the targeted preparation and isolation of 1,2,4-trithiolanes, 1,2,4,5-tetrathianes and 1,2,3,5,6-pentathiepanes is described in Chem. Lett. p 1517-1520 (1988**).** Starting from 2,4,6-trisubstituted 5,6-dihydro-1,3,5-dithiazines, 3,6-disubstituted 1,2,4,5-tetrathianes were prepared by oxidation with N-bromo- or N-chloro-succinimide. In a further reaction, these 1,2,4,5-tetrathianes were converted into the corresponding 1,2,4-trithiolanes or 1,2,3,5,6-pentathiepanes by desulphonation or by sulphonation. However, it seems questionable whether 3,6- dimethyl-1,2,4,5-tetrathiane was actually isolated, because a boiling point of only 59 degrees centigrade is indicated for this substance, which clearly appears to be too low.

The tetrathianes of formula **2** to be used according to the invention - and the trithiolanes of formula **1** and/or pentathiepanes of formula **3** obtainable therefrom - can presumably not be obtained by means of purification by distillation owing to their high boiling points. If the synthesis procedure of the mentioned publication in Chem. Letters is followed, purification by filtration over silica gel does not seem to be suitable either, because derivatives of N-chloro- or N-bromo-succinimide cannot be separated off completely thereby and the prepared tetrathianes of formula 2, on account of this contamination, are accordingly unsuitable for use in flavourings or in semi-finished or finished products suitable for consumption.

When the synthesis procedure in Chem. Letters is followed, the oxidative agent for the preparation of the tetrathianes of formula 2 is bromine generated in situ from N-bromosuccinimide. It has now been shown that, as an alternative, tetrathianes of formula 2 can be prepared using an oxidation system in which bromine is prepared in situ by the comproportionation reaction of a bromate and a bromide. The advantage is that the secondary components from the oxidising agent are purely inorganic in nature and can readily be separated off. After purification by chromatography, the tetrathianes of formula **2** are obtainable as pure sub stances, as are the pentathiepanes of formula **3** and the trithiolanes of formula **1.** Further details are given in the section "Synthesis".

### Sensory properties

Own investigations of the applicant resulted in the sensory properties indicated herein below; the following conditions were chosen for internal tastings: substance to be tasted from about 300 ppb to about 100 ppm, in each case in 5 percent sugar solution and in 0.5 percent sodium chloride solution. The sensory evaluation on the basis of the indicated descriptors was in each case made on a scale of from 1 (very weak) to 9 (very strong).

By way of example, the sensory properties of the tetrathianes of formula **2** are indicated using the example of 3,6-diethyl-1,2,4,5-tetrathiane (formula 2, R³ and R⁴ = ethyl) (purity greater than 95 percent), which is particularly preferred according to the invention:
leek (7), shiitake (5), onion (4), green (3), steamed (3), sulphurous (2), burnt (2).
3,6-Diethyl-1,2,4,5-tetrathiane is a very intensive (7) tasting, adherent (7) sub stance of great richness (7). 3,6-Diethyl-1,2,4,5-tetrathiane is capable of imparting an odour or taste impression of fresh (raw) or boiled (steamed) Allium plants, in particular of leek and onion, as well as of shiitake.

The taste of shiitake is generally described as spicy-aromatic, leek-like and reminiscent of garlic.

The comments made in respect of 3,6-diethyl-1,2,4,5-tetrathiane apply in the same or a similar manner to other tetrathianes of formula 2.

The interesting sensory profile of the tetrathianes of formula 2, in particular of 3,6-diethyl-1,2,4,5-tetrathiane, is particularly valuable because, although strong leek and onion impressions are predominant, an unpleasant and undesirable sulphurous note is largely absent (see the above evaluation). Because green aspects are also present, tetrathianes of formula 2 impart impressions of freshly cut leek and onions. Owing to the marked shiitake note, the sensory profile has a very complex effect and permits use in a broad spectrum of applications and flavouring compositions.

Despite these properties, which are already interesting and valuable in themselves, the sensory profile of the tetrathianes of formula 2 can be further supplemented and completed according to the invention by combination with the trithiolanes of formula **1** and/or pentathiepanes of formula **3** indicated above.

It is particularly advantageous that the mixtures according to the invention are able to impart the sensory impression of Allium plants, especially of onion, shallot, garlic, chives, leek, wild garlic, as well as shiitake and/or durian, without imparting a marked sulphurous odour or taste impression.

Surprisingly, fragrance, flavouring and taste-imparting substance compositions and preparations comprising such compositions frequently experience more balanced taste impressions by the addition of the tetrathianes of formula **2** to be used according to the invention or of the mixtures according to the invention; the fragrance, flavouring and taste-imparting substance compositions and the preparations acquire more body and the sensory profile is more rounded, more complex, more authentic and/or richer.

The tetrathianes of formula **2** to be used according to the invention, or the mixtures according to the invention (as defined above), which comprise at least one tetrathiane of formula **2** and at least one trithiolane of formula 1 and/or pentathiepane of formula **3,** are particularly suitable for incorporation into flavouring com positions and preparations having the taste orientations meat, fish or seafood, in particular of pork, beef, lamb, chicken, shrimp, prawn, crab and squid.

A mixture comprising 35 % b.w. 3,5-diethyl-1,2,4-trithiolane, 28 % b.w. 3,6-diethyl- 1,2,4,5-tetrathiane and 35 % b.w. 4,7-diethyl-1,2,3,5,6-pentathiepane has been evaluated in sensory terms, the evaluation being carried out as above:
onion (6), leek (6), shiitake (4), steamed (4), roasted (3), green (3), durian (3), sulphurous (3), earthy (2).

This very intensive (7) tasting, adherent (7) mixture exhibited great richness (6) and had a markedly more balanced, more rounded sensory profile than the individual compounds. In particular, a stronger impression of freshly cut onion and freshly cut leek is to be detected, mixed with boiled (steamed) and roasted aspects, accompanied by shiitake notes and slightly fruity nuances.

A similar sensory impression as with the (pure) tetrathianes of formula **2** is observed in the case of the (pure) pentathiepanes of formula 3, with the difference that the sulphurous impression is markedly stronger (5-6) and foul (3-4) and earthy (2-3) notes, also notes that are fruity (2-3) when first tasted, are observed, such as, for example, in the case of 4,7-diethyl-1,2,3,5,6-pentathiepane (formula 3, R⁵ and R⁶ = ethyl) or 4,7-diisopropyl-1,2,3,5,6-pentathiepane (formula 3, R⁵ and R⁶ = isopropyl). In mixtures according to the invention, R¹ to R⁶ particularly preferably represent the same C₂-C₄-alkyl radical, but combinations of the following type, for example, are also advantageous: 3,5-diethyl-1,2,4-trithiolane, 3-methyl-6-propyl-1, 2,4,5- tetrathiane and 4-isobutyl-7-methyl-1,2,3,5,6-pentathiepane.

When a suitable mixing ratio is established, a mixture according to the invention (as defined above) imparts the fundamental sensory impressions (primary impressions) of onion, shallot, garlic, chives, leek, durian and shiitake. By varying the amounts or the mixing ratios, the person skilled in the art (flavourist) can provide these primary impressions with fresh (raw), boiled, steamed, fried, roasted or fatty and fried notes.

In mixtures according to the invention, the weight ratio compound of formula **2:** compound of formula 1 is preferably in the range from 1: 3 to 10: 1, preferably in the range from 1: 2 to 5: 1, particularly preferably in the range from 1: 2 to 2: 1. The above-mentioned sensory effects apply in particular to these preferred mixtures.

In the same manner, the sensory effects mentioned above apply to preferred mixtures according to the invention in which the weight ratio compound of formula **2:** compound of formula **3** is in the range from 1: 20 to 20: 1, preferably in the range from 1: 10 to 10: 1, particularly preferably in the range from 1: 5 to 5: 1, very particularly preferably in the range from 1: 3 to 3: 1.

A particularly preferred mixture according to the invention comprises
(i) from 5 to 95 % b.w., preferably from 10 to 70 % b.w., particularly preferably from 15 to 60 % b.w., of at least one compound of formula **2** as well as
(ii-a) from 2 to 95 % b.w., preferably from 10 to 70 % b.w., particularly preferably from 15 to 60 % b.w., of at least one compound of formula **1** and/or
(ii-b) from 1 to 95 % b.w., preferably from 10 to 70 % b.w., particularly preferably from 15 to 60 % b.w., of at least one compound of formula **3,**
the sum of the compounds **1** and (**2** and/or **3**) being from 75 to 100 % b.w., preferably from 90 to 100 % b.w., based on the total weight of the mixture. Particular preference is given to mixtures that comprise compounds of formulae 1, 2 and 3, the sum of the compounds 1, 2 and 3 again preferably being from 90 to 100 percent. Particular preference is given to mixtures comprising
(i) from 15 to 40 % b.w. of a compound of formula **2,**
(ii-a) from 15 to 40 % b.w. of a compound of formula **1** and
(ii-b) from 15 to 40 % b.w. of a compound of formula **3**
the sum of the compounds 1, 2 and 3 being from 90 to 100 % b.w., based on the total weight of the mixture.

The invention relates also to a method of producing, enhancing or modifying a taste and/or odour which corresponds to that of an Allium plant, comprising the step: mixing a sensorial effective amount of a mixture according to the invention (as described above, preferably in a preferred form) with a base preparation.

The tetrathianes of formula **2** and the mixtures according to the invention may be in undiluted or diluted form, for example in the form of solutions, and can be stored in that form. The diluents or solvents used are preferably water, methanol, ethanol, propylene glycol, glycerol, acetone, dichloromethane, diethyl ether, hexane, heptane, triacetin, vegetable oil or fats, such as, for example, vegetable triglyceride (optionally kosher), supercritical carbon dioxide, or mixtures of the mentioned diluents or solvents.

It has been found that the tetrathianes of formula **2** and the mixtures according to the invention, when diluted (that is to say when in contact with an inert diluent), are durable and stable to storage for a surprisingly long time and, when diluted, for example at room temperature (about 20 degrees centigrade), do not change in sensory terms over a prolonged period (at least 24 months). A content of tetrathiane of formula **2** or of mixture according to the invention in the range of from 0.01 to 30 % b.w., preferably from 0.1 to 10 % b.w., particularly preferably from 0.5 to 5 % b.w., based on the mixture comprising diluent and tetrathiane of formula **2** or mixture according to the invention, is advantageous.

Particularly suitable for dilution are
(a) triacetin and
(b) the triglycerides of medium chain length (C₆ to C₁₀; MCT = medium-chain triglycerides) having identical or different fatty acid radicals, which triglycerides are substantially neutral in terms of taste.

It is further surprising that fragrance, flavouring and taste-imparting substance compositions, and preparations and semi-finished products comprising such compositions, retain their sensory character (e.g. fresh onions) over a prolonged period in the presence of the tetrathianes of formula 2 or of the mixtures according to the invention.

The present invention accordingly further provides preparations (in particular those for nutrition or enjoyment), semi-finished products and fragrance, flavouring and taste-imparting substance compositions which comprise
(i) a mixture according to the invention or
(ii) one or more tetrathianes of formula 2 to be used according to the invention (wherein R³, R⁴ are each independently of the other C₂-C₄-alkenyl or C₂-C₄-alkyl but R³ and R⁴ are not simultaneously ethyl).

See in this respect the following description and the accompanying claims. The tetrathianes of formula **2** to be used according to the invention, or the mixtures according to the invention, can be used as such in the preparations for nutrition or enjoyment in particular, but they are preferably used together with further flavourings or taste-imparting substances as a constituent of a flavouring composition according to the invention. In addition to the tetrathiane(s) of formula 2 (wherein R³ and R⁴ are as defined above) to be used according to the invention, or in addition to a mixture according to the invention, such flavouring compositions comprise further sensorial active substances such as, for example, synthetic, natural or nature identical flavourings or plant extracts. The ratios of
(i) the tetrathiane(s) of formula **2** or
(ii) mixtures according to the invention
to the further constituents of a flavouring composition according to the invention is naturally dependent to a great extent on the desired overall sensory impression of the flavouring composition.

### Preparations

Preparations according to the invention for nutrition or enjoyment comprise a tetrathiane of formula **2** (wherein R³ and R⁴ are as defined above), or a mixture according to the invention, in a sensorial effective amount, and one or more other basic substances, auxiliary substances and additives conventional for foodstuffs and snacks. Preparations according to the invention generally com prise from 0.0000001 % b.w. (0.001 ppm) to 0.05 % b.w. (500 ppm), preferably from 0.0000002 % b.w. (0.002 ppm) to 0.01 % b.w. (100 ppm), particularly preferably from 0.0000005 % b.w. (0.005 ppm) to 0.005 % b.w. (50 ppm) of the tetrathiane of formula **2,** or of mixture according to the invention, based on the total weight of the preparation. Further basic substances, auxiliary substances and additives conventional for foodstuffs or snacks may be present in amounts of up to 99.999999 % b.w., preferably from 10 to 80 % b.w., based on the total weight of the preparation. The preparations may further comprise water in an amount of up to 99.999999 % b.w., preferably from 5 to 80 % b.w., based on the total weight of the preparation.

The preparations for nutrition or enjoyment within the scope of the invention are, for example, baked articles (e.g. bread, dry biscuits, cakes, other baked articles), confectionery (e.g. chocolate, fruit gums, hard and soft caramels, chewing gum, liquorice), alcoholic or non-alcoholic drinks (e.g. coffee, tea, wine, drinks containing wine, beer, drinks containing beer, liqueurs, whiskies, brandies, soft drinks containing fruit, isotonic drinks, refreshment drinks, nectars, fruit and vegetable juices, fruit or vegetable juice preparations), instant drinks, meat products (e.g. ham, fresh sausage or raw sausage preparations), eggs or egg products (dried egg, egg white, egg yolk), cereal products (e.g. breakfast cereals, muesli bars), milk products (e.g. milk drinks, milk ice, yoghurt, kefir, fresh cheese, soft cheese, hard cheese, dried milk powder, whey, butter, buttermilk), fruit preparations (e.g. jams, fruit ice, fruit sauces), vegetable preparations (e.g. ketchup, sauces, dried vegetables), snack articles (e.g. baked or fried potato crisps or potato pulp products, corn- or peanut-based extrudates), products based on fat and oil or emulsions thereof (e.g. mayonnaise, remoularde, dressings), ready meals and soups, spices, spice mixtures and also, especially, seasonings, which are used in the snacks sector. The preparations within the scope of the invention may also be used as semi-finished products for the production of further preparations for nutrition or enjoyment. The preparations within the scope of the invention may also be in the form of capsules, tablets (uncoated and coated tablets, e.g. enteric coatings), dragees, granules, pellets, solids mixtures, dispersions in liquid phases, in the form of emulsions, in the form of powders, in the form of pastes or in the form of other swallowable or chewable preparations as food supplements.

The tetrathiane of formula **2** to be used according to the invention, or a mixture according to the invention, or a flavouring composition comprising these sub stances, in all use forms, can either be added via a dry, pasty or liquid pre-mix or can be added as such, without having previously been mixed with other constituents. The time of the addition is dependent on the use form and the production method.

Preferred preparations for nutrition or enjoyment comprising a tetrathiane of formula 2, or a mixture according to the invention, are soups (instant soups such as onion, leek, asparagus, pea, carrot, tomato, chicken, beef, mushroom, fish, shrimp and crab soups, as well as tinned soups of the mentioned varieties), sauces (instant sauces of the varieties beef, pork, lamb, fish, crab, shrimp, mush room, asparagus, onion and leek), spices (bouillon of the varieties beef, pork, chicken, lamb and vegetable, as well as seasoning mixtures of various types or spices of table salt), snacks or snack articles (baked or fried potato crisps or potato pulp products, corn- or peanut-based extrudates), products based on fat or oil (mayonnaise, remoulade and dressings) and ready meals.

A mixture according to the invention is preferably used in seasonings. Suitable seasonings comprise, for example, synthetic, natural or nature identical flavour ings as well as carriers such as polysaccharides, e.g. maltodextrine, salts, e.g. cooking salt, spices, e.g. paprika and pepper, sugars or sugar substitutes or sweeteners, e.g. saccharine, and taste enhancers, e.g. monosodium glutamate and/or inosine monophosphate.

The preparations according to the invention comprising a tetrathiane of formula 2 or a mixture according to the invention can be produced by incorporating the tetrathiane of formula 2, or the mixture according to the invention, in the form of a solution or in the form of a mixture with a solid or liquid carrier, into the preparations for nutrition or enjoyment. Advantageously, the preparations according to the invention, comprising a tetrathiane of formula 2 or a mixture according to the invention, in the form of solutions can be converted into a solid preparation by spray drying.

For the production of the preparations it is also possible in a further preferred embodiment for a tetrathiane of formula 2, or a mixture according to the invention, and optionally other constituents of the preparation according to the invention to be incorporated beforehand into emulsions, into liposomes, e.g. starting from phosphatidyl choline, into microspheres, into nanospheres or into capsules of a matrix suitable for foodstuffs and snacks, e.g. a matrix of starch, starch derivatives, other polysaccharides, natural fats, natural waxes or of proteins, e.g. gelatin. In a further embodiment, a tetrathiane of formula 2, or a mixture accord ing to the invention, is complexed beforehand with suitable complexing agents, for example with cyclodextrins or cyclodextrin derivatives, preferably beta - cyclodextrin, and used in that form. As other constituents of the preparations according to the invention for nutrition or enjoyment there may be used further basic substances, auxiliary substances and additives conventional for foodstuffs or snacks, for example water, mixtures of fresh or processed, vegetable or animal base or raw substances (e.g. raw, roast, dried, fermented, smoked and/or boiled meat, egg, bone, cartilage, fish, crustaceans and shellfish, vegetables, fruits, herbs, nuts, vegetable or fruit juices or pastes or mixtures thereof), digestible or non-digestible carbohydrates (e.g. sucrose, maltose, fructose, glucose, dextrins, amylose, amylopectin, inulin, xy- lans, cellulose), sugar alcohols (e.g. sorbitol, mannitol, xylitol), natural or hardened fats (e.g. tallow, lard, palm oil, coconut fat, hardened vegetable fat), fatty oils (e.g. sunflower oil, groundnut oil, maize oil, thistle oil, olive oil, walnut oil, fish oil, soybean oil, sesame oil), fatty acids or their salts (e.g. potassium stearate, potassium palmitate), proteinogenic or non-proteinogenic amino acids and related compounds (e.g. taurine, creatine, creatinine), peptides, natural or processed proteins (e.g. gelatine), enzymes (e.g. peptidases, glucosidases, lipases), nucleic acids, nucleotides (inositol phosphate), taste-modulating substances (e.g. sodium glutamate, 2-phenoxypropionic acid), emulsifiers (e.g. lecithins, diacyl-glycerols), stabilisers (e.g. carrageenan, alginate, locust bean flour, guar flour), preservatives (e.g. benzoic acid, sorbic acid), antioxidants (e.g. tocopherol, ascorbic acid), chelators (e.g. citric acid), organic or inorganic acidifying agents (e.g. malic acid, acetic acid, citric acid, tartaric acid, phosphoric acid), bitter sub stances (e.g. quinine, caffeine, limonine), sweeteners (e.g. saccharine, cyclamate, aspartame, neotame, neohesperidine dihydrochalcone), mineral salts (e.g. sodium chloride, potassium chloride, magnesium chloride, sodium phosphates), substances that prevent enzymatic browning (e.g. sulphite, ascorbic acid), essential oils, plant extracts, natural or synthetic colourings or colouring pigments (e.g. carotinoids, flavonoids, anthocyanins, chlorophyll and derivatives thereof), spices, as well as fragrances, synthetic, natural or nature identical flavourings and taste-imparting substances.

The invention relates also to the use of the preparations according to the invention as semi-finished products for the flavouring of preparations produced there from as finished products. Finally, the invention relates also to a process for the preparation of a mixture according to the invention, comprising the following steps: oxidation of a 2,4,6-trisubstituted 5,6-dihydro-1,3,5-dithiazine with one or more oxidising agents from the group consisting of: oxygen, chlorine, bromine and iodine, the oxidising agent being used in elemental form or being prepared in situ from inorganic precursors. The oxidising agent used is preferably bromine, which is advantageously prepared in situ from bromide and bromate.

### Synthesis

### Synthesis of the trithiolanes of formula 1, tetrathianes of formula 2 and pentathiepanes of formula 3

As already mentioned, the preparation of 3,6-dimethyl-1,2,4,5-tetrathiane described in Chem. Lett. Vol. 1517-1520 (1988) is not optimal. The use of N-bromo- or N- chloro-succinimide and the reaction conditions (in methylene chloride, -78°C, 5 hours) are disadvantageous; even after purification of the 3,6-dimethyl-1, 2,4,5- tetrathiane, traces of the succinimide derivative used can still be detected.

It has now been found that it is possible to oxidise 2,4,6-trisubstituted 5,6- dihydro-1,3,5-dithiazines using the oxidising agents oxygen, chlorine, bromine or iodine, with bromine being preferred. It is particularly preferred to generate the bromine in situ from sodium bromate and sodium bromide.

The oxidation yields a product mixture which, depending on the reaction conditions, comprises different proportions of trithiolane of formula 1, tetrathiane of formula 2 and pentathiepane of formula 3, see the schematic reaction equation below, in which the individual radicals R represent a C₂-C₄-alkyl or -alkenyl radical. The radicals R* and R** are in many cases identical with R, but this is not absolutely necessary.

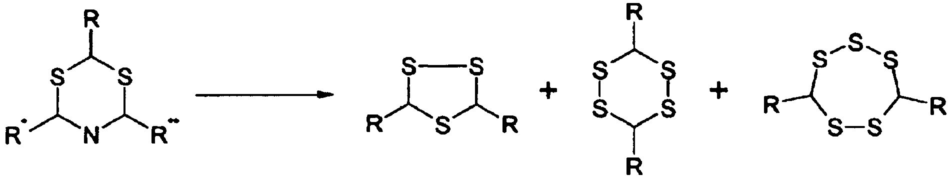

For the preparation of products with mixed substituents, see the "Representative Example" herein below.

The pH value of the reaction mixture during the oxidation of the 2,4,6- trisubstituted 5,6-dihydro-1,3,5-dithiazines is adjusted to a pH value of from 0 to 6, preferably from 0 to 3, particularly preferably from 0.5 to 1.5, by addition of acids; a pH value of about 1 is most preferred.

Preferred acids for adjusting the pH value are nitric acid, sulphuric acid, hydrochloric acid, phosphoric acid, formic acid, acetic acid, oxalic acid, citric acid, p- toluenesulfonic acid, as well as polymer-carried acids or resins, such as are commercially available, for example, under the names Amberlyst or Lewatit. Hydrochloric acid and sulphuric acid are particularly preferred.

The reaction is carried out in a diluent. In addition to water there may be used as diluent organic, optionally aqueous, solvents.

Suitable organic diluents are, for example, acetone, methanol, ethanol, dichloromethane, trichloromethane, tetrachloromethane, ethylene dibromide, ethylene dichloride, ethyl acetate, n-pentane, n-hexane, n-heptane, cyclohexane, toluene, tetrahydrofuran, 1,4-dioxane, acetonitrile, 1-propanol, tert. -butyl methyl ether and diethyl ether.

Preferred organic (optionally aqueous) diluents are methanol, ethanol, tetrahydrofuran and 1,4-dioxane and mixtures thereof with water. Particularly preferred diluents are tetrahydrofuran and mixtures of tetrahydrofuran and water. Within the scope of the working up, the reaction product comprising the com pounds of formulae 1 to 3 is converted into an organic phase that is not (no longer) miscible with water by phase separation and/or by extraction of the aqueous phase with a water-immiscible solvent, such as, for example, diethyl ether or tert. -butyl methyl ether. Ethanol, for example, is added to that organic phase, and the readily volatile, water-immiscible solvent is removed by distillation. An oily crude product separates out from the resulting e.g. ethanolic solution and is purified, preferably by filtration over adsorbents.

The adsorbents are preferably activated carbon, silica gel having many different degrees of activity, neutral aluminium oxide having many different degrees of activity, basic aluminium oxide having many different degrees of activity, Celite or sodium sulfate. The use of neutral aluminium oxide having many different degrees of activity is preferred.

The purified product mixture can be used as such for the applications according to the invention. The individual compounds trithiolane of formula **1,** tetrathiane of formula **2** and pentathiepane of formula **3** can, if required, be isolated from the mixture by preparative chromatography.

### Examples

### Representative Example

Preparation of a mixture of 3,5-diethyl-1,2,4-trithiolane, 3,6-diethyl-1,2,4,5-tetrathiane and 4,7-diethyl-1,2,3,5,6-pentathiepane

Unless indicated otherwise, all amounts are by weight.

### a) 2,4,6-Triethyl-1,3,5-dithiazinane (= 2,4,6-triethyl-5,6-dihydro-l,3,5-dithiazine)

400 ml of 20 percent ammonium sulphide solution and 250 ml of tetrahydrofurane (THF) were mixed. 40 g of propanal were added in metered amounts at room temperature, and the mixture was stirred for 4 hours at room temperature. After addition of 100 ml of methyl tert.-butyl ether (MTBE), the phases were separated and the aqueous phase was extracted twice with MTBE. After washing with water and drying over sodium sulfate, the solvent was removed in vacuum. 47 g of crude product having a content of the desired 2,4,6-triethyl-1,3,5-dithiazinane of 96 percent were obtained. The product was used in the next synthesis step without being worked up further,

### b) Oxidation of the 2,4,6-triethyl-1,3,5-dithiazinane

50 g of 2,4,6-triethyl-1,3,5-dithiazinane from step a) were dissolved in 400 ml of THF, and a solution of 38 g of sodium bromate and 26 g of sodium bromide in 400 ml of water was added thereto. The pH value of the solution was then brought carefully to about pH 1 by the controlled addition of 1 N hydrochloric acid, while cooling with ice. When the reaction had subsided, the ice bath was re moved and stirring was carried out for 3 hours at room temperature. The reaction mixture was extracted twice with diethyl ether and the combined organic phases were washed in succession with sodium hydrogen carbonate solution and so dium thiosulfate solution and then dried over sodium sulfate. After filtration, the solution was diluted with ethanol and the ether was removed carefully in vacuum.

10 g of an oily phase separated out of the ethanolic solution. This crude product contained 18 percent 3,5-diethyl-1,2,4-trithiolane, 11 percent 3,6-diethyl-1,2,4,5-tetrathiane and 14 percent 4,7-diethyl-1,2,3,5,6-pentathiepane.

The crude product was taken up in ether and filtered over neutral aluminium oxide with ether/pentane. After removal of the solvent in vacuum, 4 g of a mixture according to the invention having the following composition (determination by GC) were obtained:
35 % 3,5-diethyl-1,2,4-trithiolane,
28 % 3,6-diethyl-1,2,4,5-tetrathiane,
35 % 4,7-diethyl-1,2,3,5,6-pentathiepane

The individual substances could be isolated by preparative chromatography⁴ with a purity of greater than 95 percent in each case.
⁴HPLC conditions: column = Gram Saphir 65 C18, phase = 5 micro m 125*8 mm, eluant = ethanol/water = 70:30

The structures of formulae **1, 2** and **3** could be confirmed by comparing their Raman spectra with those of known reference compounds. The Raman spectra of known reference compounds show that S-S disulphide vibrations have characteristic positions in the Raman spectrum in the range from 540 to 500 cm^{"1} and S- S-S trisulphide vibrations have characteristic positions in the range from 510 to 450 cm^{"1}. There may be mentioned by way of example diisopropyl disulphide with S-S vibrations at 524 and 510 cm^{"1}, 1,2,5,6-tetrathiocyclooctane with S-S vibrations at 521 and 504 cm^{"1}, diisopropyl trisulphide with S-S-S vibrations at 507 and 487 cm^{"1} and 1,2,3,5,6-pentathiepane with S-S vibration at 502 cm^{"1} or S-S-S vibrations at 485 and 465 cm^{"1}.

In the respective Raman spectra, 3,5-diethyl-1,2,4-trithiolane showed a S-S vibration at 518 cm^{"1}, 3,6-diethyl-1,2,4,5-tetrathiane showed S-S vibrations at 533 and 514 cm^{"1}, and 4,7-diethyl-1,2,3,5,6-pentathiepane showed S-S vibrations at 526 and 507 cm^{"1} or S-S-S vibrations at 493, 479 and 465 cm^{"1}.

The meaning of the radicals in the dithiazine according to step a) and the trithiolanes, tetrathianes and pentathiepanes according to step b) is determined by the choice of the aldehyde(s) used in step a).

If, for example, a molar equivalent amount of a mixture of propanal and isobutyraldehyde is used in step a) instead of the propanal, then there are formed in addition to 2,4,6-triethyl-1,3,5-dithiazinane and in addition to 2,4,6-triisopropyl- 1,3,6-dithiazinane also 1,3,5-dithiazinanes having mixed alkyl radicals (ethyl and isopropyl radicals), from which there are obtained, after the oxidation according to step b), the corresponding trithiolanes of formula 1, tetrathianes of formula 2 and pentathiepanes of formula 3 having mixed alkyl radicals.

### Application Example 1

### Modification of a typical leek flavouring mixture

A typical leek flavouring (= mixture A) could be prepared by the following composition: a mixture of propanethiol (0.05 %), diallyl disulphide in 1 % vegetable triglyceride (0.10 %), hexanal (0.15 %), leek oil (0.20 %), dipropyl trisulfide (0.40 %), 2-trans-hexenal (0.60 %), 1-penten-3-ol (1.20 %), allinate (allyl isothiocyanate) (1.60 %), 3- cis-hexenol (1.71 %) and dipropyl disulphide (2.01 %) was mixed in a matrix such as vegetable triglyceride (91.98 %).
1. To mixture A so obtained there was added from 0.1 to 0.5 weight % of a 1 % solution of 3,6-diisopropyl-1,2,4,5-tetrathiane in vegetable triglyceride. The flavouring composition so obtained (mixture A1) exhibited more complex notes, whereby particular mention may be made of interesting nuances of roast onion with pleasant fatty notes.
2. A 1 % solution in vegetable triglyceride of a mixture according to the invention comprising 29 % 3,5-diethyl-1,2,4-trithiolane, 39 % 3,6-diethyl-1, 2,4,5- tetrathiane and 31 % 4,7-diethyl-1,2,3,5,6-pentathiepane was prepared. From 0.1 to 0.5 weight % of this 1 % solution was added to mixture A. The flavouring composition so obtained (mixture A2) exhibited markedly more body, naturalness and authenticity as well as more complexity, so that the taste profile of the natural leek was perceived as more rounded and more harmonious as a result of the added fresh notes.

### Application Example 2

### Modification of a typical onion flavouring mixture

A typical onion flavouring (= mixture B) could be prepared by the following recipe: a mixture of 2-trans-hexenal (0.26 %), leek oil (0.31 %), allinate (0.39 %), methyl propyl disulfide (0.51 %), foetida oil (0.65 %), dithiazine in vegetable triglyceride (1.28 %), dimethyl trisulfide (1.28 %), dipropyl disulfide (2.56 %), dipropyl trisulfide (5.13 %), propylene glycol (8.20 %) and onion oil (10.26 %) was dissolved in a matrix such as triacetin (69.17 %).
1. 0.2 weight % of a 1 % solution of 3,6-diethyl-1,2,4,5-tetrathiane in vegetable triglyceride was added to mixture B. The flavouring composition so obtained (mixture B1) exhibited overall more richness and natural, leek-like notes.
2. A 1 % solution in vegetable triglyceride of a mixture according to the invention comprising 35 % 3,5-diethyl-1,2,4-trithiolane, 28 % 3,6-diethyl-1, 2,4,5- tetrathiane and 35 % 4,7-diethyl-1,2,3,5,6-pentathiepane was prepared. From 0.1 to 0.5 weight % of this 1 % solution was added to mixture B.

The flavouring composition so obtained (mixture B2) exhibited a considerably more marked characteristic of a freshly cut onion. This effect cannot be produced by the onion oil contained in mixture B alone. Likewise, pleasant roasted notes are found in mixture B2. The sensory impression of a freshly cut onion was long-lasting as a result of the addition of the mentioned mixture according to the invention and was (sensorial) stable for a prolonged period. Particularly noteworthy is the durability of the taste impression of freshly cut onion, because onion oil loses freshness when stored.

### Application Example 3

### Durian flavouring mixture

A typical durian flavouring (= mixture C) could be prepared by the following rec ipe: a mixture of 10 % caproic acid in propylene glycol (0.10 %), methylcyclopen- tenolone-3,2,2 nat. (0.10 %), ethyl isovalerate (0.51 %), dimethyl sulfide (0.20 %), 10 % diacetyl nat. in propylene glycol (0.20 %), 10 % ethyl maltol in propylene glycol (0.51 %), 1 % phenylethyl acetate in propylene glycol (0.81 %), vanillin (1.01 %), isopropylthiol (1.01 %), butyl acetate (1.01 %), ethyl butyrate (1.01 %), ethyl acetate (1.01 %) and propanethiol (2.02 %) was dissolved in a matrix such a propylene glycol (90.70 %).

A 1 % solution in vegetable triglyceride of a mixture according to the invention comprising 35 % 3,5-diethyl-1,2,4-trithiolane, 28 % 3,6-diethyl-1,2,4,5-tetrathiane and 35 % 4,7-diethyl-1,2,3,5,6-pentathiepane was prepared. From 0.1 to 0.6 weight % of this 1 % solution was added to mixture C. The flavouring composition so obtained exhibited a markedly stronger taste impression and higher intensity as well as a longer adherence. In addition, the durian note is significantly more complex and more natural.

### Application Example 4

### Shiitake flavouring mixture

A typical shiitake flavouring (= mixture D) can be prepared by the following rec ipe: a mixture of 3,5-dimethyl-2-ethylpyrazine (0.08 %), 2,6-dimethylpyrazine (0.15 %), 1 % methylthiopropanol in propylene glycol (0.15 %), 2,5-dimethylpyrazine (0.15 %), 3-octanol (0.45 %), methylfurfuryl disulfide (0.45 %), 0.1 % o-cresol in triacetin (0.76 %), diisopropyl trisulfide (1.21 %), 2-hydroxy-3-methyl-cyclopent-2- enone nat. (1.37 %), 1-octen-3-ol (1.52 %), dimethyl trisulfide (2.27 %), 1 % di- methyloxyfuron in vegetable triglyceride (2.27 %) and 10 % trithiahexane-2,3,5 in triacetin (4.55 %) is dissolved in a matrix such as vegetable triglyceride (84.62 %).

By addition of from 0.1 to 0.8 weight % of a 1 % solution of 3,6-diethyl-1,2,4,5- tetrathiane in triacetin to mixture D, the earthy, spicy nuances of a boiled shiitake mushroom are enhanced.

A 1 % solution in vegetable triglyceride of a mixture according to the invention comprising 35 % 3,5-diethyl-1,2,4-trithiolane, 28 % 3,6-diethyl-1,2,4,5-tetrathiane and 35 % 4,7-diethyl-1,2,3,5,6-pentathiepane was prepared. From 0.1 to 0.6 weight % of this 1 % solution was added to mixture C. The complex sensory impression of the natural taste profile of a shiitake mushroom could thus be prepared in an outstanding manner.

### Application Example 5

### Snack articles

Snacks are mostly savoury snacks, such as, for example, potato corn crisps, extrudates, pellets, popcorn, pretzels, and dough products baked in fat or in the oven. The application of a tetrathiane of formula **2,** or of a mixture according to the invention, or of a flavouring composition comprising one of these substances, to a snack article can be carried out by way of seasonings, sprayed-on oil slurry, fatty filling or dough flavouring.

Example of a basic recipe for the production of crackers: wheat flour (60-63 %), baking powder (1.0-1.5 %), vegetable fat (6.0-6.5 %), maltose syrup (2.0-2.5 %), emulsifier (1.2-1.8 %), ammonium bicarbonate (1.5-2.0 %), dried skimmed milk powder (1.0-1.5 %), fresh baker's yeast (0.3-0.9 %), table salt (0.3-0.6 %), water (20.0-23.5 %).

The onion flavouring composition from Application Example 2 (mixture B2) was added in an amount of from 0.05 to 5 % to the basic recipe, metered addition in an amount in the range of from 0.1 to 2 %, based on the weight of the basic recipe, preferably being carried out.

### Application Example 6

### Seasoning

The preparation of seasonings, for example for snacks, was carried out accord ing to the following recipe.

Example of a basic recipe for the preparation of seasonings:
Table salt (10-25 %), carrier (e.g. whey powder) (40-60 %), fillers (e.g. powdered fat) (5-15 %), taste enhancer (1.5-3.5 %), auxiliary substance (e.g. silica) (0.1-5 %), cheese powder (10-30 %), hydrolysed vegetable proteins (5-10 %), yeast extract (5-15 %), spices (1-5 %), acidifying agent (e.g. citric acid) (0.1-1.0 %), colouring (e.g. paprika extract) (0.1-1.0 %). The onion flavouring composition from Application Example 2 (mixture B2) was added in an amount of from 1 to 20 % to the basic recipe, metered addition in an amount in the range of from 5 to 15 weight %, based on the weight of the basic recipe, preferably being carried out.

### Application Example 7

### Cream of leek soup

The preparation of a cream of leek soup was carried out according to the following recipe.

Example of a basic recipe for the preparation of a cream of leek soup:
Milk fat component, Vana Crema (25-30 %), potato starch (15-25 %), milk sugar, lactose (18-22 %), maltodextrine (10-12 %), salt (7-9 %), glutamate monosodium salt (2-4 %), vegetable fat (2-4 %), powdered spinach (1-2 %), citric acid powder (0.2-0.4 %), powdered leeks (1-2 %), freeze-dried leek pieces (about 10x10 mm) (0.5-1.5 %), vegetable broth powder (0.2-0.5 %), Curcuma extract (0.05-0.1 %)

The leek flavouring composition from Application Example 1 (mixture A2) was added in an amount of from 1 to 15 weight % to the basic recipe, the metered addition of from 3 to 10 weight %, based on the weight of the basic recipe, preferably being carried out.

## Claims

1. Mixture comprising a compound of formula **2** and a compound of formula **1** as well as, optionally, a compound of formula **3** 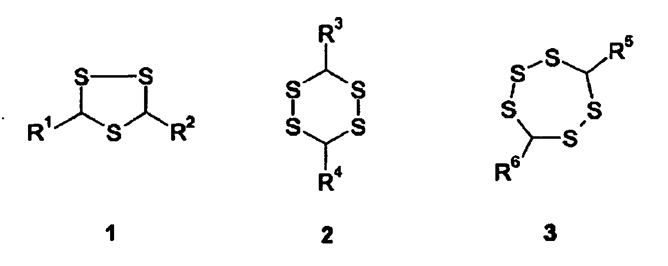 wherein R¹, R², R³, R⁴, R⁵ and R⁶ independently of one another represent a C₂-C₄-alkyl or -alkenyl radical and wherein the weight ratio compound of formula **2 :** compound of formula **1** is greater than or equal to 1: 5.

2. Mixture comprising a compound of formula **2** and a compound of formula **3** as well as, optionally, a compound of formula **1** 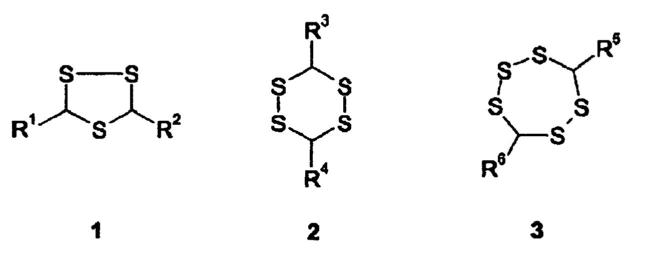 wherein R¹, R², R³, R⁴, R⁵ and R⁶ independently of one another represent a C₂-C₄-alkyl or -alkenyl radical.

3. Mixture according to Claim 1 or 2, wherein the weight ratio compound of formula **2:**
compound of formula **1** is in the range from 1: 3 to 10: 1.

4. Mixture according to any one of Claims 1 to 3, wherein the weight ratio compound of formula **2:** compound of formula **3** is in the range from 1: 20 to 20: 1.

5. Mixture according to any one of Claims 1 to 4, comprising
(i) from 5 to 95 % b.w. of at least one compound of formula **2** as well as
(ii-a) from 2 to 95 % b.w. of at least one compound of formula **1** and/or
(ii-b) from 1 to 95 % b.w. of at least one compound of formula **3,**
the sum of the compounds 1 and (2 and/or 3) being from 75 to 100 % b.w. based on the total weight of the mixture.

6. Mixture according to Claim 5, comprising
(i) from 15 to 40 % b.w. of a compound of formula 2,
(ii-a) from 15 to 40 % b.w. of a compound of formula 1 and
(ii-b) from 15 to 40 % b.w. of a compound of formula 3
the sum of the compounds **1, 2** and **3** being from 90 to 100 % b.w., based on the total weight of the mixture.

7. Method of producing, enhancing or modifying a taste and/or odour which corresponds to that of an Allium plant, comprising the step: mixing a sensorial effective amount of a mixture according to any one of claims 2 to 7 with a base preparation.

8. Fragrance, flavouring or taste-imparting substance composition comprising
(i) one or more compounds of formula **2** 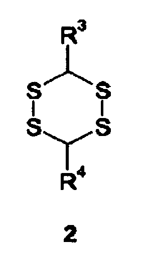 wherein R³, R⁴ each independently of the other is C₂-C₄-alkenyl or C₂-C₄-alkyl but R³ and R⁴ are not simultaneously ethyl, or
(ii) a mixture according to any one of Claims 2 to 7.

9. Preparation comprising a mixture according to any one of Claims 1 to 6 or a fragrance, flavouring or taste-imparting substance composition according to Claim 8.

10. Process for the preparation of a mixture according to any one of Claims 1 to 6, comprising the following steps: oxidation of a 2,4,6-trisubstituted 5,6-dihydro-i,3,5-dithiazine with one or more oxidising agents from the group consisting of: oxygen, chlorine, bromine and iodine, the oxidising agent being used in elemental form or being prepared in situ from inorganic precursors.

11. Process according to Claim 10, wherein the oxidising agent used is bromine, which is prepared in situ from bromide and bromate.

## Patentansprüche

1. Mischung, umfassend eine Komponente der Formel **2** und eine Komponente der Formel **1** sowie, optional, eine Komponente der Formel **3** 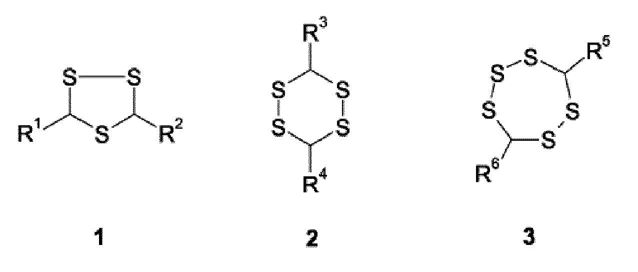 worin R¹, R², R³, R⁴, R⁵ und R⁶ unabhängig voneinander ein C₂-C₄-Alkyl oder -Alkenyl radikal darstellen und worin das Gewichtsverhältnis der Komponente der Formel **2 :** Komponente der Formel **1** größer oder gleich zu 1: 5 ist.

2. Mischung, umfassend eine Komponente der Formel **2** und eine Komponente der Formel **3** sowie, optional, eine Komponente der Formel **1** 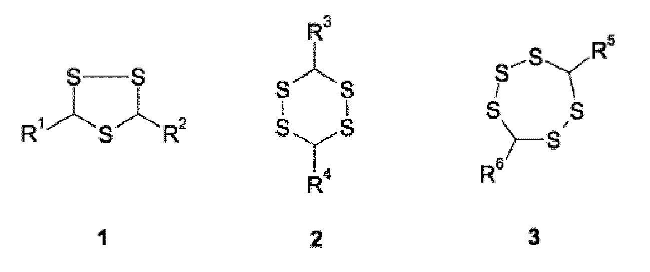 worin R¹, R², R³, R⁴, R⁵ und R⁶ unabhängig voneinander ein C₂-C₄-Alkyl oder -Alkenyl radikal darstellen.

3. Mischung nach Anspruch 1 oder 2, worin das Gewichtsverhältnis der Komponente der Formel **2 :** Komponente der Formel **1** in einem Bereich von 1: 3 bis 10: 1 ist.

4. Mischung nach einem der Ansprüche 1 bis 3, worin das Gewichtsverhältnis der Komponente der Formel **2:** Komponente der Formel **3** in einem Bereich von 1: 20 bis 20: 1 ist.

5. Mischung nach einem der Ansprüche 1 bis 4, umfassend
(i) von 5 bis 95 Gew.% mindestens einer Komponente der Formel **2,** sowie
(ii-a) von 2 bis 95 Gew.% mindestens einer Komponente der Formel **1** und/oder
(ii-b) von 1 bis 95 Gew.% mindestens einer Komponente der Formel **3,**
die Summe der Komponenten 1 und (2 und/oder 3) ist von 75 bis 100 Gew.%, bezogen auf das Gesamtgewicht der Mischung.

6. Mischung nach Anspruch 5, umfassend
(i) von 15 bis 40 Gew.% einer Komponente der Formel 2,
(ii-a) von 15 bis 40 Gew.% einer Komponente der Formel 1 und
(ii-b) von 15 bis 40 Gew. % einer Komponente der Formel 3,
die Summe der Komponenten **1, 2** und **3** ist von 90 bis 100 Gew.% bezogen auf das Gesamtgewicht der Mischung.

7. Verfahren zur Erzeugung, Steigerung oder Modifizierung eines Geschmacks und/oder Geruchs, das mit dem von der Pflanze Allium korrespondiert, umfassend die Schritte: mischen einer sensorisch effektiven Menge einer Mischung nach einem der Ansprüche 2 bis 7 mit einer Basiszusammensetzung.

8. Duft-, Aroma- oder geschmacksvermittelnde Substanz-Zusammensetzung, umfassend
(i) eine oder mehrere Komponenten der Formel **2** 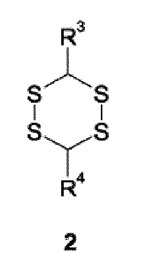 worin R³, R⁴ jedes unabhängig voneinander C₂-C₄-Alkenyl oder C₂-C₄-Alkyl ist, jedoch R³ und R⁴ sind nicht gleichzeitig Ethyl, oder
(ii) eine Mischung nach einem der Ansprüche 2 bis 7.

9. Zusammensetzung, umfassend eine Mischung nach einem der Ansprüche 1 bis 6 oder ein Duft-, Aroma- oder geschmacksvermittelnde Substanz-Zusammensetzung nach Anspruch 8.

10. Verfahren zur Herstellung einer Mischung nach einem der Ansprüche 1 bis 6, umfassend die folgenden Schritte: Oxidation von 2,4,6-trisubstituted 5,6-dihydro-1,3,5-dithiazine mit einem oder mehreren oxidierenden Agentien aus der Gruppe bestehend aus: Sauerstoff, Chlor, Brom und Iod, das oxidierende Agens wird in elementare Form verwendet oder wird in situ aus anorganischen Prekursorn hergestellt.

11. Verfahren nach Anspruch 10, wobei das oxidierende Agens Brom ist, welches in situ aus Bromiden und Bromaten hergestellt wird.

## Revendications

1. Mélange comprenant un composé de formule 2 et un composé de formule 1 ainsi qu'éventuellement un composé de formule 3 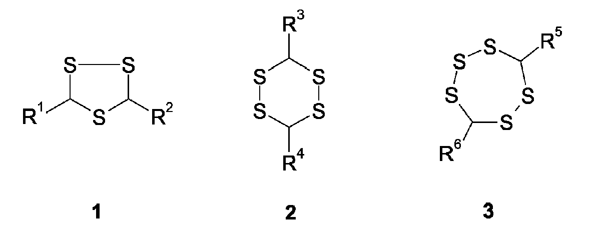 dans lesquelles R¹, R², R³, R⁴, R⁵ et R⁶ représentent, indépendamment les uns des autres, un radical alkyle ou alcényle en C₂-C₄ et dans lesquelles le rapport pondéral du composé de formule 2 au composé de formule 1 est supérieur ou égal à 1:5.

2. Mélange comprenant un composé de formule 2 et un composé de formule 3 ainsi qu'éventuellement un composé de formule 1 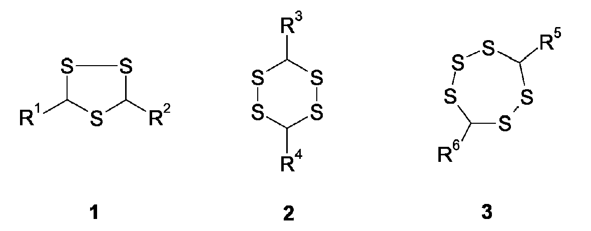 dans lesquelles R¹, R², R³, R⁴, R⁵ et R⁶ représentent, indépendamment les uns des autres, un radical alkyle ou alcényle en C₂-C₄.

3. Mélange selon la revendication 1 ou 2, dans lequel le rapport pondéral du composé de formule 2 au composé de formule 1 se trouve dans la plage allant de 1:3 à 10:1.

4. Mélange selon l'une quelconque des revendications 1 à 3, dans lequel le rapport pondéral du composé de formule 2 au composé de formule 3 se trouve dans la plage allant de 1:20 à 20:1.

5. Mélange selon l'une quelconque des revendications 1 à 4, comprenant
(i) de 5 à 95% en poids d'au moins un composé de formule 2, ainsi que
(ii-a) de 2 à 95% en poids d'au moins un composé de formule 1, et/ou
(ii-b) de 1 à 95% en poids d'au moins un composé de formule 3,
la somme des composés 1 et (2 et/ou 3) allant de 75 à 100% en poids sur la base du poids total du mélange.

6. Mélange selon la revendication 5, comprenant
(i) de 15 à 40% en poids d'un composé de formule 2,
(ii-a) de 15 à 40% en poids d'un composé de formule 1, et
(ii-b) de 15 à 40% en poids d'un composé de formule 3,
la somme des composés 1, 2 et 3 allant de 90 à 100% en poids sur la base du poids total du mélange.

7. Méthode de production, d'amélioration ou de modification d'un goût et/ou d'une odeur qui correspond à celui ou celle d'une plante d'*Allium,* comprenant l'étape consistant à mélanger une quantité efficace sur le plan sensoriel d'un mélange selon l'une quelconque des revendications 2 à 7 avec une préparation de base.

8. Composition de substance conférant un goût, un arôme, ou un parfum, comprenant
(i) un ou plusieurs composés de formule 2 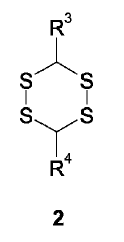 dans laquelle R³ et R⁴ représentent chacun, indépendamment l'un de l'autre, un radical C₂-C₄-alcényle ou C₂-C₄-alkyle, mais R³ et R⁴ ne représentent pas simultanément éthyle, ou
(ii) un mélange selon l'une quelconque des revendications 2 à 7.

9. Préparation comprenant un mélange selon l'une quelconque des revendications 1 à 6 ou une composition de substance conférant un goût, un arôme, ou un parfum selon la revendication 8.

10. Procédé de préparation d'un mélange selon l'une quelconque des revendications 1 à 6, comprenant les étapes suivantes : l'oxydation d'une 5,6-dihydro-1,3,5-dithiazine 2,4,6-trisubstituée par un ou plusieurs agents oxydants choisis dans le groupe constitué par l'oxygène, le chlore, le brome et l'iode, l'agent oxydant étant utilisé sous forme élémentaire ou étant préparé *in situ* à partir de précurseurs inorganiques.

11. Procédé selon la revendication 10, dans lequel l'agent oxydant utilisé est le brome, qui est préparé *in situ* à partir de bromure et de bromate.
